(19) Europäisches Patentamt
European Patent Office
Office européen des brevets

(11) Veröffentlichungsnummer: **0 005 240**
**B1**

(12) **EUROPÄISCHE PATENTSCHRIFT**

(45) Veröffentlichungstag der Patentschrift: **29.04.81**

(21) Anmeldenummer: **79101258.6**

(22) Anmeldetag: **26.04.79**

(51) Int. Cl.³: **C 07 D 231/20,**
C 07 D 231/24,
A 01 N 43/56

(54) **N,N-Dimethyl-O-pyrazolyl-carbaminsäureester, Verfahren zu ihrer Herstellung und ihre Verwendung als Insektizide.**

(30) Priorität: **06.05.78 DE 2819932**

(43) Veröffentlichungstag der Anmeldung:
**14.11.79 Patentblatt 79/23**

(45) Bekanntmachung des Hinweises auf die Patenterteilung:
**29.04.81 Patentblatt 81/17**

(84) Benannte Vertragsstaaten:
**BE CH DE FR GB IT NL**

(56) Entgegenhaltungen:
**DE - A - 1 542 661**
**DE - A - 2 644 588**
**DE - A - 2 644 589**

(73) Patentinhaber: **BAYER AG**
**Zentralbereich Patente, Marken und Lizenzen**
**D-5090 Leverkusen 1, Bayerwerk (DE)**

(72) Erfinder: **Maurer, Fritz, Dr.**
**Röberstrasse 8**
**D-5600 Wuppertal 1 (DE)**
Erfinder: **Riebel, Hans-Jochem, Dr.**
**Heimatstrasse 1**
**D-5418 Selters (DE)**
Erfinder: **Schröder, Rolf, Dr.**
**Pahlkestrasse 17**
**D-5600 Wuppertal 1 (DE)**
Erfinder: **Hammann, Ingeborg, Dr.**
**Belfortstrasse 9**
**D-5000 Köln (DE)**
Erfinder: **Homeyer, Bernhard, Dr.**
**Obere Strasse 28**
**D-5090 Leverkusen 3 (DE)**

**EP 0 005 240 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

N,N-Dimethyl-O-pyrazolyl-carbaminsäureester, Verfahren zu ihrer Herstellung und ihre Verwendung als Insektizide

Die Erfindung betrifft neue N,N-Dimethyl-O-pyrazolyl-carbaminsäureester, mehrere Verfahren zu ihrer Herstellung sowie ihre Verwendung als Insektizide.

Es ist bereits bekannt, daß N,N-Dimethyl-O-pyrazolyl-carbaminsäureester, wie z.B. N,N-Dimethyl-O-(1-phenyl-3-methyl-pyrazol(5)yl)- und N,N-Dimethyl-O-(1-iso-propyl-3-methyl-pyrazol(5)yl)-carbaminsäureester, insektizide Eigenschaften aufweisen (vergleiche CH-PS 279 553 - Chem, Abstracts 47 (1953), 10172 a).

Aus DE-OS 2 644 588 sind N,N-Dimethyl-O-1-methyl-thioalkyl-pyrazol (5)yl -carbaminsäureester bekannt. Die Verbindungen sind zum Teil toxisch und zum Teil nicht ausreichend in ihrer Dauerwirkung.

Es wurden die neuen N,N-Dimethyl-O-pyrazolylcarbaminsäure ester der Formel I

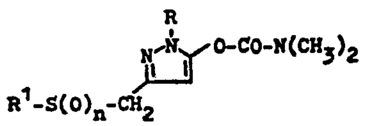

(I)

in welcher

R für Wasserstoff oder gegebenenfalls substituiertes Alkyl,

$R^1$ für Alkyl und

n für 1 oder 2 steht,

gefunden.

Die neuen Verbindungen zeichnen sich durch starke insektizide Wirksamkeit aus.

Weiter wurde gefunden, daß man die neuen N,N-Dimethyl-O-pyrazolyl-carbaminsäureester der Formel I erhält, wenn man

a) Hydroxy-pyrazole der Formel

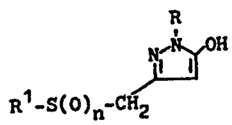

(II)

worin

R, $R^1$ und n die oben angegebene Bedeutung haben, mit N,N-Dimethylcarbaminsäurehalogeniden der Formel

$$Hal—CO—N(CH_3)_2 \qquad (III)$$

worin

Hal für Chlor oder Brom steht,

gegebenenfalls in Gegenwart eines Säureakzeptors und gegebenenfalls in Gegenwart eines Verdünnungsmittels umsetzt oder

b) Hydroxypyrazole der Formel II

worin R, $R^1$ und n die oben angegebene Bedeutung haben,

mit Phosgen und anschließend mit Dimethylamin gegebenenfalls in Gegenwart eines Säureakzeptors und gegebenenfalls in Gegenwart eines Verdünnungsmittels umsetzt,

c) N,N-Dimethyl-O-pyrazol(5)yl-carbaminsäureester der Formel I.

worin R und $R^1$ die oben angegebene Bedeutung haben und

n für Null steht,

mit der äquimolaren Menge des Oxidationsmittels Wasserstoffperoxid, gegebenenfalls in Gegenwart eines Verdünnungsmittels umsetzt oder

d) N,N-Dimethyl-O-pyrazol(5)yl-carbaminsäureester der Formel I,

worin R und $R^1$ die oben angegebene Bedeutung haben und

n für Null steht,

mit wenigstens zwei Äquivalenten des Oxidationsmittels m-Chlor-perbenzoesäure, gegebenenfalls in Gegenwart eines Verdünnungsmittels umsetzt.

Überraschenderweise zeigen die erfindungsgemäßen N,N-Dimethyl-O-pyrazolyl-carbaminsäureester eine bessere insektizide Wirkung als die entsprechenden aus dem Stand der Technik vorbekannten Verbindungen analoger Konstitution und gleicher Wirkungsrichtung. Die Produkte gemäß vorliegender Erfindung stellen somit eine echte Bereicherung der Technik dar.

Bevorzugt sind Verbindungen der Formel I in welcher R, R[1] und n die bei den Ausgangsprodukten der Verfahren a—d angegebenen vorzugsweisen Bedeutungen besitzen (s. unten).

Verwendet man nach Verfahren (a) 1-Methyl-3-äthylsulfinylmethyl-5-hydroxy-pyrazol und N,N-Dimethyl-carbaminsäure-chlorid, nach Verfahren (c) N,N-Dimethyl-O-(1-methyl-3-äthylthiomethyl-pyrazol(5)yl-carbaminsäureester und Wasserstoffperoxid, nach Verfahren (d) N,N-Dimethyl-O-(1-propyl-3-äthylthiomethyl-pyrazol(5)yl-carbaminsäureester und nach Verfahren (b) 1-tert.-Butyl-3-methylsulfinyl-methyl-5-hydroxy-pyrazol, Phosgen und Dimethylamin als Ausgangsstoffe, so können die Reaktionsabläufe durch folgende Formelschemata wiedergegeben werden:

a)
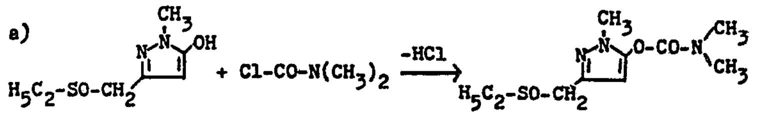


b)
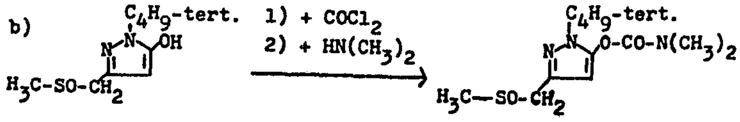


c)
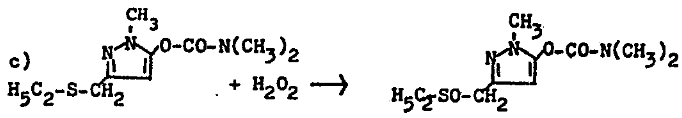


d)
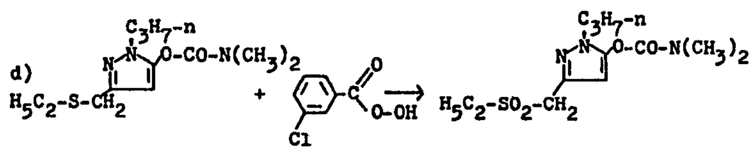

Die bei Verfahren (a) und (b) als Ausgangsstoffe zu verwendenden Hydroxy-pyrazole sind durch Formel II definiert. Vorzugsweise stehen darin

R für Wasserstoff sowie für geradkettiges oder verzweigtes Alkyl mit 1 bis 8, insbesondere 1 bis 5 Kohlenstoffatomen und für Cyanäthyl,

R[1] für geradkettiges Alkyl mit 1 bis 5, insbesondere 1 bis 3 Kohlenstoffatomen und

n für 1 oder 2.

Die Hydroxy-pyrazole der Formel II erhält man durch Umsetzung von Hydrazin oder Hydrazinderivaten mit $\gamma$-Alkylthioacetessigsäureestern bei 0 bis 40°C in inerten Verdünngsmitteln und anschließende Oxidation der dabei erhaltenen 3-Alkylthiomethyl-5-hydroxy-pyrazole mit Wasserstoffperoxid oder m-Chlorperbenzoesäure, ebenfalls bei 0 bis 40°C und ebenfalls in Gegenwart von Verdünnungsmitteln.

Als Beispiele für die erfindungsgemäß zu verwendenden Hydroxy-pyrazole seien genannt:

3-Methylsulfinylmethyl-, 3-Äthylsulfinylmethyl- und 3-n-Propylsulfinylmethyl-5-hydroxy-pyrazol, 1-Methyl-3-methylsulfinylmethyl-, 1-Methyl-3-äthylsulfinylmethyl- und 1-Methyl-3-n-propyl-sulfinyl-methyl-5-hydroxy-pyrazol, 1-Äthyl-3-methylsulfinylmethyl-, 1-Äthyl-3-äthylsulfinylmethyl- und 1-Äthyl-3-n-propyl-sulfinylmethyl-5-hydroxy-pyrazol,

1-n-Propyl-3-methylsulfinylmethyl-, 1-n-Propyl-3-äthylsulfinylmethyl- und 1-n-Propyl-3-n-propyl-sulfinylmethyl-5-hydroxy-pyrazol,

1-iso-Propyl-3-methylsulfinylmethyl-, 1-iso-Propyl-3-äthylsulfinylmethyl- und 1-iso-Propyl-3-n-propyl-sulfinylmethyl-5-hydroxy-pyrazol,

1-n-Butyl-3-methylsulfinylmethyl-, 1-n-Butyl-3-äthylsulfinylmethyl- und 1-n-Butyl-3-n-propyl-sulfinyl-methyl-5-hydroxy-pyrazol,

1-iso-Butyl-3-methylsulfinylmethyl-, 1-iso-Butyl-3-äthylsulfinylmethyl- und 1-iso-Butyl-3-n-propyl-sulfinylmethyl-5-hydroxy-pyrazol,

1-sek.-Butyl-3-methylsulfinylmethyl-, 1-sek.-Butyl-3-äthylsulfinylmethyl- und 1-sek.-Butyl-3-n-propyl-sulfinylmethyl-5-hydroxy-pyrazol,

1-n-Amyl-3-methylsulfinylmethyl-, 1-n-Amyl-3-äthylsulfinylmethyl- und 1-n-Amyl-3-n-propyl-sulfinyl-methyl-5-hydroxy-pyrazol,

1-iso-Amyl-3-methylsulfinylmethyl-, 1-iso-Amyl-3-äthylsulfinylmethyl- und 1-iso-Amyl-3-n-propyl-sulfinylmethyl-5-hydroxy-pyrazol,

1-sek.-Amyl-3-methylsulfinylmethyl-, 1-sek.-Amyl-3-äthylsulfinylmethyl- und 1-sek.-Amyl-3-n-propyl-sulfinylmethyl-5-hydroxy-pyrazol sowie

1-(2-Cyano-äthyl)-3-methylsulfinylmethyl-,

1-(2-Cyano-äthyl)-3-äthylsulfinylmethyl- und
1-(2-Cyano-äthyl)-3-n-propylsulfinylmethyl-5-hydroxy-pyrazol.

Als Carbaminsäurehalogenid der Formel III wird bei Verfahren (a) vorzugsweise das nach literaturbekannten Verfahren herstellbare und häufig in der Technik verwendete N,N-Dimethyl-carbaminsäurechlorid eingesetzt.

Die bei den Verfahren (c) und (d) als Ausgangsstoffe einzusetzenden N,N-Dimethyl-O-(3-alkylthiomethylpyrazol)(5)yl)-carbaminsäureester sind durch Formel I mit der Maßgabe, daß n für Null steht, definiert. Sie können analog Verfahren (a) aus den entsprechenden 3-Alkylthiomethyl-5-hydroxy-pyrazolen und N,N-Dimethyl-carbaminsäurechlorid hergestellt werden.

Vorzugsweise werden in Verfahren (c) und (d) Verbindungen der Formel I, in der n für Null steht, und worin

R für Wasserstoff und geradkettiges oder verzweigtes Alkyl mit 1 bis 8, insbesondere 1 bis 5 Kohlenstoffatomen sowie für Cyanoäthyl steht und

$R^1$ für geradkettiges Alkyl mit 1 bis 5, insbesondere 1 bis 3 Kohlenstoffatomen steht,

als Ausgangsstoffe eingesetzt.

Als Beispiele seien genannt:
O-(1-Methyl-3-methylthiomethyl-pyrazol(5)yl)-,
O-(1-Methyl-3-äthylthiomethyl-pyrazol(5)yl)-,
O-(1-Methyl-3-n-propylthiomethyl-pyrazol(5)yl)-,
O-(1-Äthyl-3-methylthiomethyl-pyrazol(5)yl)-,
O-(1-Äthyl-3-äthylthiomethyl-pyrazol(5)yl)-,
O-(1-Äthyl-3-n-propylthiomethyl-pyrazol(5)yl)-,
O-(1-n-Propyl-3-methylthiomethyl-pyrazol(5)yl)-,
O-(1-n-Propyl-3-äthylthiomethyl-pyrazol(5)yl)-,
O-(1-n-Propyl-3-n-propylthiomethyl-pyrazol(5)yl)-,
O-(1-iso-Propyl-3-methylthiomethyl-pyrazol(5)yl)-,
O-(1-iso-Propyl-3-äthylthiomethyl-pyrazol(5)yl)-,
O-(1-iso-Propyl-3-n-propylthiomethyl-pyrazol(5)yl)-,
O-(1-n-Butyl-3-methylthiomethyl-pyrazol(5)yl)-,
O-(1-n-Butyl-3-äthylthiomethyl-pyrazol(5)yl)-,
O-(1-n-Butyl-3-n-propylthiomethyl-pyrazol(5)yl)-,
O-(1-iso-Butyl-3-methylthiomethyl-pyrazol(5)yl)-,
O-(1-iso-Butyl-3-äthylthiomethyl-pyrazol(5)yl)-,
O-(1-iso-Butyl-3-n-propylthiomethyl-pyrazol(5)yl)-,
O-(1-sek.-Butyl-3-methylthiomethyl-pyrazol(5)yl)-,
O-(1-sek.-Butyl-3-äthylthiomethyl-pyrazol(5)yl-,
O-(1-sek.-Butyl-3-n-propylthiomethyl-pyrazol(5)yl)-,
O-(1-n-Amyl-3-methylthiomethyl-pyrazol(5)yl)-,
O-(1-n-Amyl-3-äthylthiomethyl-pyrazol(5)yl)-,
O-(1-n-Amyl-3-n-propylthiomethyl-pyrazol(5)yl)-,
O-(1-iso-Amyl-3-methylthiomethyl-pyrazol(5)yl)-,
O-(1-iso-Amyl-3-äthylthiomethyl-pyrazol(5)yl)-,
O-(1-iso-Amyl-3-n-propylthiomethyl-pyrazol(5)yl)-,
O-(1-sek.-Amyl-3-methylthiomethyl-pyrazol(5)yl)-,
O-(1-sek.-Amyl-3-äthylthiomethyl-pyrazol(5)yl)-,
O-(1-sek.-Amyl-3-n-propylthiomethyl-pyrazol(5)yl)-,
O-(1-(2-Cyano-äthyl)-3-methylthiomethyl-pyrazol(5)yl)-,
O-(1-(2-Cyano-äthyl)-3-äthylthiomethyl-pyrazol(5)yl)-,
O-(1-(2-Cyano-äthyl)-3-n-propylthiomethyl-pyrazol(5)yl)- sowie O-(3-Methylthiomethyl-pyrazol(5)yl)-,
O-(3-Äthylthiomethyl-pyrazol(5)yl)- und
O-(3-n-Propylthiomethyl-pyrazol(5)yl)-N,N-dimethylcarbaminsäureester.

Die in Verfahren (a) und (b) verwendeten Oxidationsmittel Wasserstoffperoxid und m-Chlorperbenzoesäure sind allgemein bekannte Verbindungen und können nach literaturbekannten Verfahren hergestellt werden.

Die Verfahren (a), (b), (c) und (d) zur Herstellung der neuen N,N-Dimethyl-O-pyrazolyl-carbaminsäureester werden bevorzugt unter Verwendung von Verdünngsmitteln durchgeführt. Als solche kommen praktisch alle inerten organischen Lösungsmittel infrage.

Hierzu gehören insbesondere aliphatische und aromatische gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Für Verfahren (c) werden aliphatische Carbonsäuren, wie z.B. Essigsäure, als Lösungsmittel bevorzugt.

Verfahren (a) und (b) werden bevorzugt unter Verwendung von Säureakzeptoren durchgeführt.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die erfindungsgemäßen Verfahren werden im allgemeinen bei Temperaturen zwischen 0 und 80°C durchgeführt. Bevorzugt wird für Verfahren (a) der Bereich zwischen 20 und 60°C, für Verfahren (b), (c) und (d) der Bereich zwischen 5 und 25°C.

Die Umsetzungen werden im allgemeinen bei Normaldruck durchgeführt.

Zur Durchführung von Verfahren (a) und (b) werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt. Nach Reaktionsende gießt man die Mischung in Wasser und schüttelt mit einem organischen Lösungsmittel, z.B. Toluol aus. Die organische Phase wird dann wie üblich durch Waschen, Trocknen und Abdestillieren des Lösungsmittels aufgearbeitet.

Bei Verfahren (c) werden die Reaktionspartner ebenfalls bevorzugt in äquimolaren Mengen eingesetzt. Die in der Regel hierbei als Verdünnungsmittel verwendete Essigsäure wird nach Reaktionsende im Vakuum abdestilliert. Danach gibt man ein organisches Lösungsmittel, z.B. Methylenchlorid zu und arbeitet die organische Phase wie üblich durch Waschen, Trocknen und Abdestillieren des Lösungsmittels auf.

Bei Verfahren (d) wird die als Oxidationsmittel verwendete m-Chlor-perbenzoesäure gewöhnlich im Überschuß eingesetzt, und zwar zwischen 2 und 3 Mol je Mol O-(3-Alkyl-thiomethyl-pyrazol(5)yl)-N,N-dimethyl-carbaminsäureester. Die Umsetzung wird gewöhnlich in einem mit Wasser nicht mischbaren Lösungsmittel durchgeführt. Man wäscht dann neutral und arbeitet wie unter (a) und (c) beschrieben auf.

Die neuen Verbindungen fallen zum Teil in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Soweit die neuen Produkte nach dem Abdestillieren des Lösungsmittels in fester Form anfallen, werden sie durch Umkristallisation gereinigt. Zur Charakterisierung dient der Schmelzpunkt.

Wie bereits mehrfach erwähnt, zeichnen sich die erfindungsgemäßen N,N-Dimethyl-O-pyrazolyl-carbaminsäureester durch eine hervorragende insektizide Wirkung aus.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hyginesektor vorkommen. Sie sind gegen normal sensible und resistente Arten sowie gegen alle oder einezine Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophaus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp. Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis

flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hotulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktoionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methyl-cellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch

eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießenns (pour-on and spot-on) und des Einpuders sowie durch parenterale Anwendung in Form beispielsweise der Injektion.

Beispiel A

Myzus-Test

Lösungsmittel: 3 Gewichtsteile Dimethylformamid

Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Blattläuse abgetötet wurden; 0% bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 2, 14, 8, 10, 3, 1, 6, 5, 4, 7, 16, 9.

Beispiel B

Grenzkonzentrations-Test / Wurzelsystemische Wirkung

Testinsekt: Myzus persicae

Lösungsmittel: 3 Gewichtsteile Aceton

Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinhelt Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 4, 5, 6, 14.

Beispiel C

Grenzkonzentrations-Test / Wurzelsystemische Wirkung

Testinsekt: Phaedon cochleariae-Larven

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinhelt Boden, welche in ppm (=mg/l) angegeben wird. Man füllt den behandelten Boden

in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 6.

Herstellungsbeispiele

Beispiel 1:

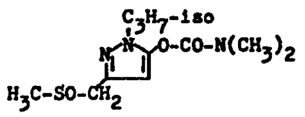

Eine Suspension aus 10,1 g (50 m Mol) 1-iso-Propyl-3-methyl-sulfinylmethyl-5-hydroxy-pyrazol, 8,4 g (60 m Mol) gemahlener Pottasche und 200 ml Acetonitril läßt man eine Stunde bei 50°C rühren, kühlt sie anschließend auf Raumtemperatur ab und fügt 5,4 g (50 m Mol) N,N-Dimethyl-carbaminsäurechlorid hinzu. Nach einstündigem Rühren bei 50°C wird die Reaktionslösung mit 200 ml Wasser versetzt und mit 300 ml Toluol ausgeschüttelt. Die organische Phase wird über Magnesiumsulfat getrocknet, filtriert und das Solvens am Rotationsverdampfer im Vakuum abgezogen. Es verbleiben 11,8 g (87% der Theorie) N,N-Dimethyl-O-(1-iso-propyl-3-methylsulfinyl-methyl-pyrazol(5)yl)-carbaminsäureester in Form schwach violetter Kristalle mit dem Schmelzpunkt 69°C.

Beispiel 2:

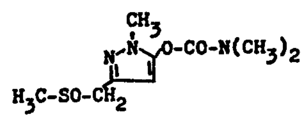

Eine Lösung von 11,5 g (0,05 Mol) N,N-Dimethyl-O-(1-methyl-3-methylthiomethyl-pyrazol(5)yl)-carbaminsäureester in 50 ml Eisessig wird bei 5—10°C mit 3,4 g (0,05 Mol) 50%igem Wasserstoffperoxid versetzt. Man rührt das Gemisch 6 Stunden bei Raumtemperatur nach und destilliert dann das Lösungsmittel im Vakuum ab. Der Rückstand wird in 100 ml Methylenchlorid gelöst und mit einer Lösung von 10 g Kaliumcarbonat in 15 ml Wasser gewaschen. Die organische Phase wird abgetrennt und über Natriumsulfat getrocknet. Dann destilliert man das Lösungsmittel im Vakuum ab. Man erhält so 12 g (98% der Theorie) N,N-Dimethyl-O-(1-methyl-3-methyl-sulfinylmethyl-pyrazol(5)yl - carbaminsäureester in Form eines braunen Öles mit dem Brechungsindex $n_D^{21}$: 1,5362.

Beispiel 3:

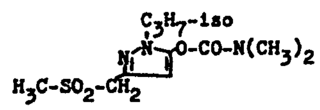

Zu einer Lösung von 12,9 g (0,05 Mol) N,N-Dimethyl-O-(1-iso-propyl-3-methylthiomethyl-pyrazol(5)yl)-carbaminsäureester in 50 ml Chloroform tropft man bei 5°C eine Lösung von 21,3 g m-Chlorperbenzoesäure in 150 ml Chloroform. Das Gemisch wird über Nacht bei Raumtemperatur nachgerührt und dann filtriert. Man wäscht das Filtrat mit 10 ml konz. Kaliumcarbonatlösung und trocknet es über Natriumsulfat. Dann wird das Lösungsmittel im Vakuum abgezogen. Zurück bleiben 13g (90% der Theorie) N,N-Dimethyl-O-(1-iso-propyl-3-methylsulfonylmethyl - pyrazol(5)yl) - carbaminsäureester in Form beiger Kristalle mit dem Schmelzpunkt 102°C.

Analog einem der Beispiele 1 bis 3 können die folgenden Verbindungen der Formel

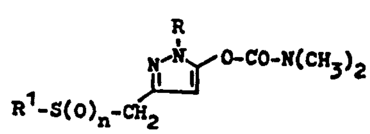

(I)

| Beispiel Nr. | R | $R^1$ | n | Ausbeute (% der Theorie) | Physikal. Daten (Brechungsindex; Schmelzpunkt °C) |
|---|---|---|---|---|---|
| 4 | $C_4H_9$-iso | $CH_3$ | 1 | 95 | $n_D^{21}$:1,5198 |
| 5 | $C_4H_9$-n | $CH_3$ | 1 | 91 | $n_D^{21}$:1,5209 |
| 6 | $C_3H_7$-iso | $C_2H_5$ | 1 | 70 | 82 |
| 7 | $C_4H_9$-sek. | $CH_3$ | 1 | 77 | $n_D^{20}$:1,5042 |
| 8 | $C_2H_5$ | $CH_3$ | 1 | 87 | $n_D^{20}$:1,5107 |
| 9 | $CH(C_2H_5)_2$ | $CH_3$ | 1 | 76 | $n_D^{20}$:1,5084 |
| 10 | $C_2H_5$ | $C_2H_5$ | 1 | 87 | $n_D^{20}$:1,5272 |
| 11 | $C_2H_5$ | $C_2H_5$ | 2 | 60 | 69 |
| 12 | $CH_3$ | $CH_3$ | 2 | 90 | 123 |
| 13 | $C_3H_7$-iso | $C_2H_5$ | 2 | 94 | 79 |
| 14 | $CH_3$ | n-$C_3H_7$ | 1 | 88 | $n_D^{20}$:1,5252 |

| Beispiel Nr. | R | $R^1$ | n | Ausbeute (% der Theorie | Physikal. Daten (Brechungsindex; Schmelzpunkt °C) |
|---|---|---|---|---|---|
| 15 | H | $CH_3$ | 1 | 61 | 80 |
| 16 | $CH_2—CH_2—CN$ | $CH_3$ | 1 | 69 | $n_D^{20}$:1,4973 |
| 17 | $CH_2—CH_2—CN$ | $C_2H_5$ | 1 | 50 | $n_D^{20}$:1,5304 |
| 18 | $CH_2—CH_2—CN$ | $CH_3$ | 2 | 85 | 102 |
| 19 | $C_2H_5$ | $CH_3$ | 2 | 85 | 82 |
| 20 | $C_4H_9$-sek. | $CH_3$ | 2 | 96 | $n_D^{20}$:1,5028 |
| 21 | $CH(C_2H_5)_2$ | $CH_3$ | 2 | | |
| 22 | $C_4H_9$-iso | $CH_3$ | 2 | 61 | 94 |
| 23 | $C_4H_9$-n | $CH_3$ | 2 | | |
| 24 | $C_4H_9$-tert. | $CH_3$ | 1 | | |
| 25 | $C_4H_9$-tert. | $CH_3$ | 2 | | |

Die als Ausgangsmaterialien zu verwendenden substituierten 5-Hydroxypyrazole können zum Beispiel wie folgt hergestellt werden:

Beispiel a:

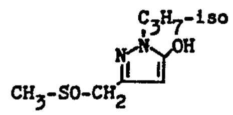

1. Stufe:

Zu einer Lösung von 13,6 g (0,11 Mol) Isopropylhydrazinsulfat in 30 ml Methanol gibt man unter Kühlen 0,11 Mol einer Lösung von Natriummethylat in Methanol. Dann versetzt man das Gemisch bei Raumtemperatur mit 17,6 g (0,1 Mol) $\gamma$-Methylthioacetessigsäureäthylester und rührt 6 Stunden nach. Anschließend wird das Lösungsmittel im Vakuum abdestilliert, der Rückstand mit Wasser verrieben und nach Kristallisation abgesaugt. Man erhält so 13,4 g (73% der Theorie) 1-Isopropyl-3-methylthiomethyl-5-hydroxypyrazol.

2. Stufe:

Zu einer Lösung von 27,2 g (0,2 Mol) dieser Verbindung in 150 ml Eisessig gibt man bei 5—10°C 13,6 g (0,2 Mol) 50%iges Wasserstoffperoxid. Man rührt das Gemisch 4 Stunden bei Raumtemperatur nach und destilliert das Lösungsmittel im Vakuum ab. Der Rückstand wird mit Äther verieben und nach Kristallisation abgesaugt. Man erhält auf diese Weise 25,8 g (64% der Theorie) 1-Isopropyl-3-methylsulfinylmethyl-5-hydroxy-pyrazol in Form eines rosa gefärbten Pulvers mit dem Schmelzpunkt 117°C.

In analoger Weise können die folgenden Verbindungen der Formel

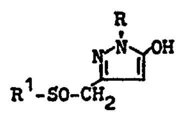

hergestellt werden:

| Beispiel | R | $R^1$ | Ausbeute (% der Theorie) | Schmelzpunkt (°C) |
|---|---|---|---|---|
| b | $C_4H_9$-iso | $CH_3$ | 39 | 114 |
| c | $C_4H_9$-n | $CH_3$ | 44 | 89 |
| d | $CH_3$ | n-$C_3H_7$ | 98 | 103 |
| e | H | $CH_3$ | 87 | 160 |
| f | $CH_2$—$CH_2$—CN | $CH_3$ | 56 | 58 |

**Patenansprüche**

1. N,N-Dimethyl-O-pyrazolylcarbaminsäureester der Formel I

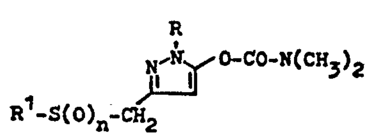

(I)

in welcher

R für Wasserstoff oder gegebenenfalls substituiertes Alkyl,

$R^1$ für Alkyl und

n für 1 oder 2 steht.

2. Verfahren zur Herstellung der N,N-Dimethyl-O-pyrazolylcarbaminsäureester der Formel I, gemäß Anspruch 1, dadurch gekennzeichnet, daß man

a) Hydroxy-pyrazole der Formel II

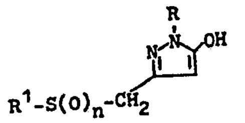

(II)

worin

R, $R^1$ und n die oben angegebene Bedeutung haben,
mit N,N-Dimethylcarbaminsäurehalogeniden der Formel III

$$Hal—CO—N(CH_3)_2 \qquad (III)$$

worin

Hal für Chlor oder Brom steht,
gegebenenfalls in Gegenwart eines Säureakzeptors und gegebenenfalls in Gegenwart eines Verdünnungsmittels umsetzt oder

b) Hydroxypyrazole der Formel II
worin R, $R^1$ und n die oben angegebene Bedeutung haben,
mit Phosgen und anschließend mit Dimethylamin gegebenenfalls in Gegenwart eines Säureakzeptors und gegebenenfalls in Gegenwart eines Verdünnungsmittels umsetzt,

c) N,N-Dimethyl-O-pyrazol(5)yl-carbaminsäureester der Formel I,
worin R und $R^1$ die oben angegebene Bedeutung haben und
n für Null steht,
mit der äquimolaren Menge des Oxidationsmittels Wasserstoffperoxid, gegebenenfalls in Gegenwart eines Verdünnungsmittels umsetzt oder

d) N,N-Dimethyl-O-pyrazol(5)yl-carbaminsäureester der Formel I.
worin R und $R^1$ die oben angegebene Bedeutung haben und
n für Null steht,
mit wenigstens zwei Aquivalenten des Oxidationsmittels m-Chlor-perbenzoesäure, gegebenenfalls in Gegenwart eines Verdünnungsmittels umsetzt.

3. Insektizide Mittel, gekennzeichnet durch einen Gehalt an mindestens einem N,N-Dimethyl-O-pyrazolylcarbaminsäureester der Formel (I) gemäß Anspruch 1.

4. Verwendung von N,N-Dimethyl-O-pyrazolylcarbaminsäureester der Formel (I) gemäß Anspruch 1 zur Bekämpfung von Insekten.

5. Verfahren zur Bekämpfung von Insekten, dadurch gekennzeichnet, daß man N,N-Dimethyl-O-pyrazolylcarbaminsäureester der Formel (I) gemäß Anspruch 1 auf Insekten und/oder ihren Lebensraum einwirken läßt.

6. Verfahren zur Herstellung insektizider Mittel, dadurch gekennzeichnet, daß man N,N-Dimethyl-O-pyrazolyl-carbaminsäureester der Formel (I) gemäß Anspruch 1 mit Streckmitteln und/oder oberflächenaktiven Mitteln vermischt.

**Claims**

1. N,N-Dimethyl-O-pyrazolylcarbamic acid esters of the formula I

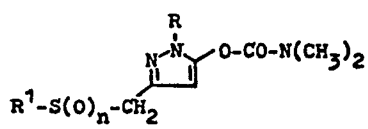

(I)

in which

R represents hydrogen or optionally substituted alkyl,
$R^1$ represents alkyl and
n represents 1 or 2.

2. Process for the preparation of the N,N-dimethyl-O-pyrazolylcarbamic acid esters of the formula I, according to Claim 1, characterised in that

a) hydroxy-pyrazoles of the formula II

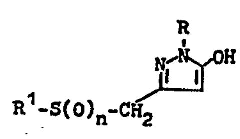

(II)

wherein

R, $R^1$ and n have the meaning indicated above, are reacted with N,N-dimethylcarbamic acid halides of the formula III

$$Hal—CO—C(CH_3)_2 \qquad (III)$$

wherein

Hal represents chlorine or bromine, if appropriate in the presence of an acid acceptor and if appropriate in the presence of a diluent, or

b) hydroxypyrazoles of the formula II,

wherein

R, $R^1$ and n have the meaning indicated above, are reacted with phosgene and the product is then reacted with dimethylamine, if appropriate in the presence of an acid acceptor and if appropriate in the presence of a diluent,

c) N,N-dimethyl-O-pyrazol-5-yl-carbamic acid esters of the formula I,

wherein

R and $R^1$ have the meaning indicated above and

n represents zero,

are reacted with the equimolar amount of the oxidising agent hydrogen peroxide, if appropriate in the presence of a diluent, or

d) N,N-dimethyl-O-pyrazol-5-yl-carbamic acid esters of the formula I,

wherein

R and $R^1$ have the meaning indicated above and

n represents zero,

are reacted with at least two equivalents of the oxidising agent m-chloro-perbenzoic acid, if appropriate in the presence of a diluent.

3. Insecticidal agents, characterised in that they contain at least one N,N-dimethyl-O-pyrazolylcarbamic acid ester of the formula (I) according to Claim 1.

4. Use of N,N-dimethyl-O-pyrazolylcarbamic acid esters of the formula (I) according to Claim 1, for combating insects.

5. Process for combating insects, characterised in that N,N-dimethyl-O-pyrazolylcarbamic acid esters of the formula (I) according to Claim 1 are allowed to act on insects and/or their environment.

6. Process for the preparation of insecticidal agents, characterised in that N,N-dimethyl-O-pyrazolylcarbamic acid esters of the formula (I) according to Claim 1 are mixed with extenders and/or surface-active agents.

**Revendications**

1. Esters de O-pyrazolyle d'acide N,N-diméthyl-carbamique de formule I:

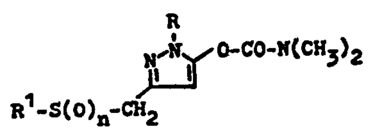

(I)

dans laquelle

R est l'hydrogène ou un groupe alkyle éventuellement substitué,

$R^1$ est un groupe alkyle et

n est égal à 1 ou 2.

2. Procédé de production d'esters de O-pyrazolyle d'acide N,N-diméthylcarbamique de formule I suivant la revendication 1, caractérisé en ce qu'on fait réagir:

a) des hydroxy-pyrazoles de formule II:

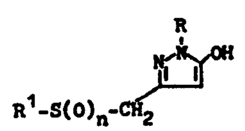

(II)

dans laquelle

R, $R^1$ et

n ont la définition donnée ci-dessus, avec des halogénures d'acide N,N-diméthylcarbamique de formule III:

$$Hal—CO—N(CH_3)_2 \qquad (III)$$

dans laquelle

Hal est le chlore ou le brome,

le cas échéant en présence d'un accepteur d'acide et en la présence éventuelle d'un diluant ou

b) des hydroxypyrazoles de formule II

dans laquelle R, $R^1$ et n ont la définition donnée ci-dessus, avec le phosgène puis avec la diméthylamine, le cas échéant en présence d'un accepteur d'acide et en la présence éventuelle d'un diluant,

c) un ester de O-pyrazol(5)yle d'acide N,N-diméthylcarbamique de formule I:

dans laquelle

R et $R^1$ ont la définition indiquée ci-dessus et

n est égal à zéro,

avec la quantité équimolaire d'agent oxydant consistant en peroxyde d'hydrogène, le cas échéant en présence d'un diluant ou

d) un ester de O-pyrazol(5)yle d'acide N,N-diméthylcarbamique de formule I

dans laquelle

R et $R^1$ ont la définition donnée ci-dessus et

n est égal à zéro

avec au moins deux équivalents d'agent oxydant consistant en acide m-chloro-perbenzoique, éventuellement en présence d'un diluant.

3. Compositions insecticides, caractérisées par une teneur en au moins un ester de O-pyrazolyle d'acide N,N-diméthylcarbamique de formule I suivant la revendication 1.

4. Utilisation d'un ester de O-pyrazolyle d'acide N,N-diméthylcarbamique de formule (I) suivant la revendication 1 pour la lutte contre les insectes.

5. Procédé de lutte contre les insectes, caractérisé en ce qu'on fait agir des esters de O-pyrazolyle d'acide N,N-diméthylcarbamique de formule (I) suivant la revendication 1 sur des insectes et/ou sur leur habitat.

6. Procédé de production de compositions insecticides, caractérisé en ce qu'on mélange des esters de O-pyrazolyle d'acide N,N-diméthylcarbamique de formule (I) suivant la revendication 1 avec des diluants et/ou des agents tensio-actifs.